# EUROPÄISCHE PATENTANMELDUNG

(11) **EP 1 025 775 A1**
(43) Veröffentlichungstag der Anmeldung: **09.08.2000**
(21) Anmeldenummer: 99126031.6
(22) Anmeldetag: 27.12.1999
(51) Int. Cl.: A45D 34/00, A61L 9/12, B42D 15/02

(54) **Trägermedium für mindestens einen Duftstoff**

(30) Priorität: 05.01.1999 DE 19900141
(71) Anmelder: Essert, Gisela Rosa, 81373 München (DE)
(72) Erfinder: Essert, Gisela Rosa, 81373 München (DE)
(74) Vertreter: Dosterschill, Peter, Dr.

(57) **Zusammenfassung**

Die Erfindung betrifft ein Trägermedium (CA) für mindestens einen ersten Duftstoff, wobei das Trägermedium (CA) mit einem ersten Produkt (P1) verbindbar ist und mindestens den ersten Duftstoff enthält.

Zum Schutz des Trägermediums vor eventuellen mechanischen Beschädigungen ist erfindungsgemäß vorgesehen, daß das erste Produkt (P1) eine Aufnahmeöffnung (OP1) für das Trägermedium (CA) aufweist. Nach dem Verbrauch eines ersten Trägermediums laßt sich die Aufnahmeöffnung für die Aufnahme eines weiteren Trägermediums nutzen.

## Beschreibung

Die Erfindung betrifft ein Trägermedium für mindestens einen Duftstoff nach dem Oberbegriff des Anspruchs 1.

Es ist bekannt, Räume. Insbesondere Wohn-, Schlaf- und Arbeitsräume gezielt mit einem Geruch zu versehen, der auf Personen, die die Räume betreten bzw. die sich in den Räumen aufhalten, eine vorgebbare Wirkung erzielen soll. So ist es bekannt, Räume mit synthetischen oder natürlichen Geruchswirkstoffen zu versehen, die bei den Personen, die die Geruchsstoffe aufnehmen, ein angenehmes Gefühl bewirken sollen.

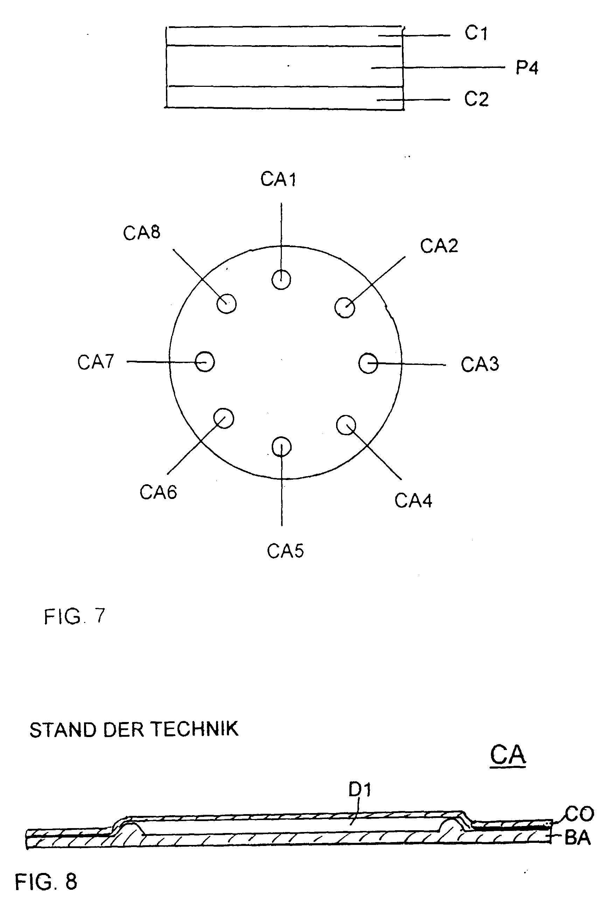

Es ist auch bereits bekannt, Parfüm-Warenproben zusammen mit Werbeschriften zu verteilen. Die Parfüm-Warenproben sind z.B. in kleinen Behältern, insbesondere in kleinen Flaschen verpackt. Weiterhin sind auch sogenannte Duftetiketten (Figur 8) bekannt, die jeweils eine geringe Menge eines beworbenen Parfüms enthalten (vgl. auch US-Patent 5,715,849; DE 296 11 416 U1; G 90 01 115.1; US-Patent 4,808,454 und Patent Abstracts of Japan 06328880 A).

Ausgehend von diesem Stand der Technik liegt der Erfindung die Aufgabe zugrunde, einem Trägermedium der eingangs genannten Art erweiterte Einsatzmöglichkeiten zuzuführen.

Diese Aufgabe wird mit einem Trägermedium gelöst, das in den Ansprüchen definiert ist.

Die Erfindung ist mit einer Mehrzahl von Vorteilen verbunden. Das erfindungsgemäße Trägermedium ist mit einem ( 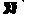 ersten") Produkt verbunden und enthält mindestens einen ( 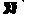 ersten") Duftstoff. Der erste Duftstoff, der sich bei einem Gebrauch des ( 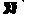 ersten") Produkts freisetzen läßt, wirkt auf die Person oder die Personen, die das Produkt gebrauchen. Das ( 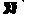 erste") Produkt ist beispielsweise eine Werbebroschüre für ein Spezialitätenrestaurant. Diese Broschüre ist mit einem erfindungsgemäßen Trägermedium, z.B. einem (zunächst versiegelten) Duftstreifen versehen, der einen Duftstoff oder eine Kombination von Duftstoffen enthalt Dieser Duftstoff bzw. diese Duftstoffe erzeugen nach ihrem Freisetzen (durch Öffnen des Duftstreifens) bei dem Leser der Werbebroschüre eine positive Grundstimmung, die mit dem Spezialitätenrestaurant bzw. mit den dort zubereiteten Spezialitäten in Verbindung gebracht wird. Der bzw. die Duftstoffe können ein z.B. appetitanregendes Gefühl erzeugen.

Die erfindungsgemäß erzielte psychische Wirkung tritt bereits wenige Sekunden nach dem Freisetzen des Duftstoffes aus dem Trägermedium ein. Duftmoleküle übertragen über die Nase mit ihren mehreren Millionen Riechzellen (verteilt auf 3 bis 4 Quadratzentimeter Nasenschleimhaut) eine Information zum sogenannten limbischen System, also dem Gehirnteil, der Gefühle wie Freude, Aufregung, Spannung, Lust auf Entspannung, Lebensenergie etc. steuert. Dort wird auch entschieden, ob etwas gut riecht bzw. welche Gefühle mit dem aufgenommenen Duft assoziiert werden.

Die mit dem erfindungsgemäßen Trägermedium erzielte Wirkung wird durch das Langzeitgedächtnis verstärkt, das mit dem Geruchsorgan gekoppelt ist.

Das erfindungsgemäße Trägermedium kann beispielsweise mit einem Druckerzeugnis (Buch, Zeitung, Zeitschrift; etc.) oder mit einem Informationsspeicher (Videoband, CD, etc.) verbunden sein. Die aus dem Trägermedium freigesetzten Duftstoffe stellen eine Assoziation zum Inhalt des Druckerzeugnisses bzw. des Informationsspeichers her. Zu einem Kochbuch werden beispielsweise Duftstoffe vorgesehen, die bestimmte Speisen oder Getränke charakterisieren oder charakteristische Duftstoffe dieser Speisen oder Getränke ergänzen (z.B. Käse/Wein bzw. Wein/Käse). Ein Videoband, das eine Landschaft am Meer zeigt, kann mit einem Duftstoff versehen werden, der salzhaltiger Luft am Meer entspricht.

Erfindungsgemäß weist das erste Produkt, z.B. ein Buchdeckel, eine Aufnahmeöffnung für das Trägermedium auf. Damit ist das Trägermedium vor eventuellen mechanischen Beschädigungen geschützt. Nach dem Verbrauch eines ersten Trägermediums laßt sich die Aufnahmeöffnung für die Aufnahme eines weiteren Trägermediums nutzen.

Weitere vorteilhafte Ausführungsformen des erfindungsgemäßen Trägermediums sind dadurch gekennzeichnet, daß das Trägermedium flüssigkeitspeicherndes Material aufweist und/oder daß das Trägermedium mit einem Duftstoff wiederbefüllbar ist. Diese Ausführungsformen zeichnen sich durch ihre Wiederverwendbarkeit und damit durch ihre umweitschonende Konstitution aus.

Eine weitere vorteilhafte Ausführungsform des erfindungsgemäßen Trägermediums ist dadurch gekennzeichnet, daß das Trägermedium eine lösbar angeordnete Abdeckung aufweist, und daß die lösbar angeordnete Abdeckung in der Weise mit einer Verpackung eines ( 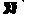 ersten") Produkts verbunden ist, daß bei einem Öffnen der Verpackung die Abdeckung gelöst wird. Damit wird der zunächst im Trägermedium enthaltene Duftstoff durch das Öffnen der Verpackung freigesetzt, ohne daß ein separates Öffnen der Trägermediumabdeckung erforderlich ist.

Die Erfindung wird nun anhand der Zeichnung beschrieben.

Es zeigt
- Figur 1: den konstruktiven Aufbau eines erfindungsgemäßen Trägermediums für mindestens einen Duftstoff;
- Figur 2: die Anordnung des Trägermediums an der Verpackung eines ersten Produkts;.
- Figur 3: die Anordnung des Trägermediums an einem ersten Produkt;
- Figur 4: ein erstes Produkt mit einer Aufnahmeöffnung für ein Trägermedium sowie das in die Aufnahmeöffnung eingeführte Trägermedium;
- Figur 5: die Anordnung eines Trägermediums an einer Verpackung eines Produkts und an diesem Produkt;
- Figur 6: die Anordnung eines Trägermediums in einem Buch;
- Figur 7: ein Behälter mit einer Mehrzahl erfindungsgemäßer Trägermedien, und
- Figur 8: ein sogenanntes Duftetikett gemäß Stand der Technik.

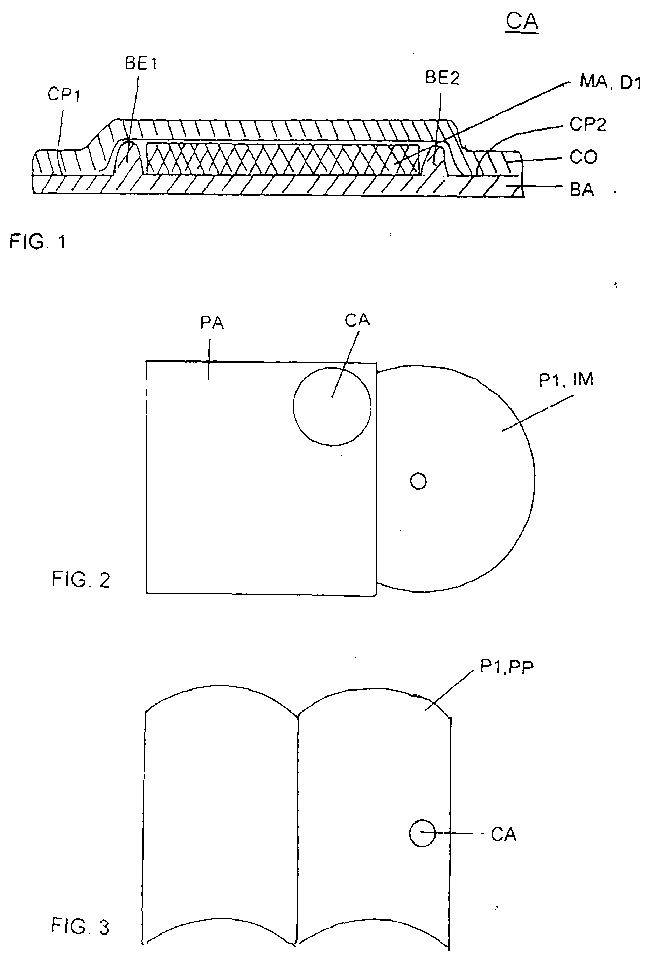

Figur 1 zeigt einen Schnitt durch ein erfindungsgemäßes Trägermedium CA. Dieses besteht aus einem Basisteil BA, einem Abdeckungsteil CO und einem Träger MA aus flüssigkeitsspeicherndem Material. Das Basisteil BA und das Abdeckungsteil CO bestehen beispielsweise aus je einer Aluminiumfolie. Beide Folien sind lösbar miteinander verbunden und insbesondere miteinander in Randbereichen CP1, CP2 des Trägermediums CA verklebt. Der Träger MA, der z.B. aus Textilmaterial, Filz, Watte oder dergleichen und als ein Kissen ausgebildet sein kann bzw. aus anderem flüssigkeitsspeicherndem Material wie beispielsweise Hydrokulturmaterial besteht, ist z.B. zwischen Begrenzungselementen BE1, BE2 eingebettet und enthält mindestens einen ersten Duftstoff D1, der noch beschrieben wird.

Das Basisteil BA kann an seiner Unterseite ein Klebemittel und damit einen selbstklebenden Bereich aufweisen, der eine Klebeverbindung an einem Produkt ermöglicht.

Die dargestellte Ausgestaltung des erfindungsgemäßen Trägermediums ermöglicht eine Wiederbefüllung mit einem oder mehreren Duftstoffen. Nach einer Wiederbefüllung wird das Abdeckungsteil CO gegen das Basisteil BA in den Bereichen CP1, CP2, die ein Klebemittel aufweisen, gedrückt und somit wiederum verschlossen.

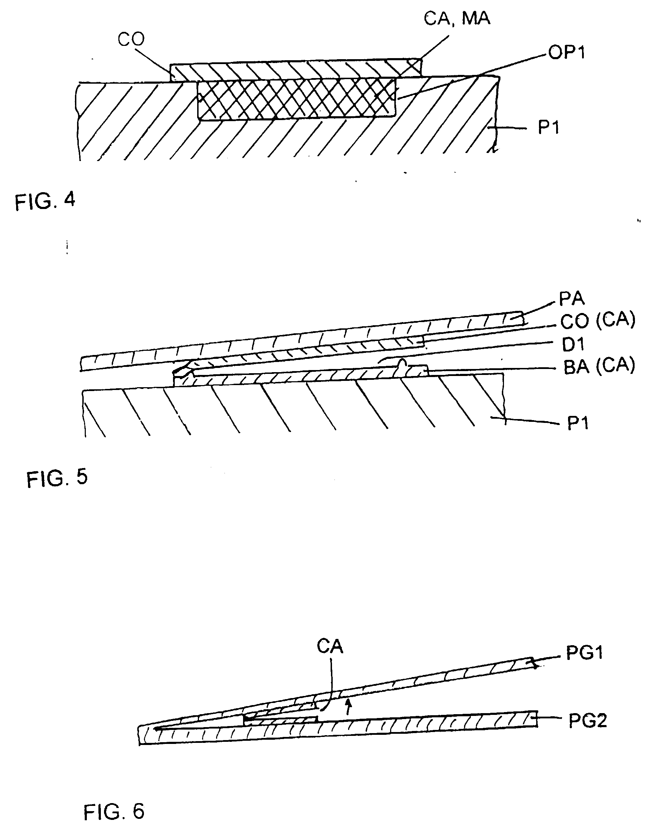

Wie noch anhand von Figur 5 beschreiben wird, kann das Abdeckungsteil CO des Trägermediums in der Weise mit einer Verpackung PA eines Produkts verbunden sein, daß bei einem Öffnen der Verpackung PA die Abdeckung bzw. das Abdeckungsteil CO gelöst wird.

Das Trägermedium kann mit alphanumerischen Zeichen bzw. mit graphischen Zeichen versehen sein, die den Duftstoff bzw. die Duftstoffe im Trägermedium bezeichnen. Das Trägermedium kann auch transparent ausgestaltet sein und/oder verschiedene geometrische Oberflächenformen, wie beispielsweise Kreise, Dreiecke, Blätter von Blumen oder Bäumen aufweisen.

Das Produkt P1, auf dem oder in dem das Trägermedium CA angeordnet wird, kann ein Druckerzeugnis PP (Figur 3) sein. Beispiele dieser Druckerzeugnisse PP sind einzelne bedruckte Blätter bzw. Seiten, Gesamtausgaben aller Art, Etiketten, sogenannte Flyers, Poster, Prospekte, Postkarten, Bücher bzw. Umschlagseiten, Beihefter, Beipackzettel und Verpackungsmaterial.
Das Druckerzeugnis PP beschreibt z.B. ein Produkt, das den ersten Duftstoff D1 und/oder einen zweiten Duftstoff D2 enthält, der im wesentlichen dem Duft des ersten Duftstoffs D1 entspricht bzw. mindestens eine Duftkomponente des ersten Duftstoffs D1 enthält.
Dieses Produkt ist beispielsweise ein Kuchen und die Duftstoffe D1, D2 sind Backaromastoffe, die den Kuchen charakterisieren.

Das Produkt P1 auf dem oder in dem das Trägermedium CA angeordnet wird, kann auch ein elektrischer und/oder ein magnetischer Informationsspeicher IM (Figur 2) sein. Beispiele dieser Informationsspeicher IM sind sogenannte Kompaktdisks, Videobänder, und Daten- bzw. Tonträger. Der Informationsspeicher IM kann Daten zu einem Produkt aufweisen, das den ersten Duftstoff D1 und/oder einen zweiten Duftstoff D2 enthält, der im wesentlichen den Duft des ersten Duftstoffs D1 entspricht bzw. mindestens eine Duftkomponente des ersten Duftstoffs D1 enthält.

Das Trägermedium CA enthält vorzugsweise einen Duftstoff D1 oder eine Kombination von Duftstoffen D1, ...Dn, wobei vorzugsweise mindestens einer der Duftstoffe ein natürlicher Duftstoff, insbesondere ein ätherisches Öl ist.

Der Duftstoff D1 bzw. mindestens ein Teil der Duftstoffe der Duftstoffkombination kann auch synthetisch sein.

Beispiele der mit dem erfindungsgemäßen Trägermedium befüllbaren Duftstoffe D1, ..., Dn sind ätherische Öle der folgenden Pflanzen:
Eichenmoos, Latschenkiefer, Riesentanne, Sandelholz, Zirbelkiefer, Zeder, Grapefruit, Mandarine, Orange, Eukalyptus, Pfefferminze, Rosmarin, Weihrauch, Vativer, Benzoe Siam, Ysop, Rosenholz, Jasmin, Bergamotte, Rosengeranie Rose, Petit Grain, Patchouli, Cistrose, Bay, Palmarosa, Litsea, Lemongrass, Douglasie, Eisenkraut, Wacholderbeere, Ylang-Ylang, Tolu, Muskatellersalbei, Cardamon, Lavendel, Koriander, Wacholder und Zimtblätter.

Mit den vorstehend genannten Duftstoffen D1, ..., Dn sind unter anderen folgende an sich bekannte Dufstoffkombinationen DK1,... DKm herstellbar und für das erfindungsgemäße Trägermedium verwendbar:
DK1
   Latschenkiefer, Riesentanne, Zeder, Eichenmoos, Wacholder;
DK2
   Grapefruit, Pfefferminze, Rosmarin;
DK3
   Weihrauch, Zeder, Riesentanne, Vativer, Benzoe Siam, Ysop;
DK4
   Zeder, Sandelholz, Rosenholz, Zirbelkiefer, Jasmin, Bergamotte, Orange, Rosengeranie;
DK5
   Rose, Petit Grain, Patchouli, Orange, Vativer;
DK6
   Rosengeranie, Cistrose, Bay, Palmarosa, Mandarine Litsea;
DK7
   Lemongrass, Grapefruit, Mandarine, Douglasie, Ysop;
DK8
   Ysop, Eukalyptus, Eisenkraut, Wacholderbeere, Lemongrass, Pfefferminze;
DK9
   Sandelholz, Patchouli, Ylang-Ylang, Orange, Eisenkraut, Bergamotte, Rosengeranie;
DK10
   Jasmin, Eichenmoos, Tolu, Sandelholz, Muskatellersalbei;
DK11
   Orange, Jasmin, Cardamon, Lavendel;
DK12
   Koriander, Zimtblätter, Ylang-Ylang, Cardamon, Tolu, Patchouli.

Die erfindungsgemäß verwendeten Duftstoffe können auch an sich bekannte Parfums sein.

Der Duftstoff D1 bzw. eine Duftstoffkombination ist ungleich einer Probe bzw. Warenprobe eines zweiten Produkts (z. B. Parfum) und kann für ein bestimmtes Unternehmen entwickelt sein, so daß das erfindungsgemäße Trägermedium in diesem Fall einen unternehmensindividuellen Duftstoff bzw. eine unternehmensindividuelle Duftstoffkombination enthält.

Wie schon beschrieben, zeigt Figur 2 die Anordnung eines selbstklebenden Trägermediums CA an der Verpackung PA einer sogenannten Kompaktdisk (Compact disk CD) P1, IM und Figur 3 zeigt die Anordnung eines selbstklebenden Trägermediums CA in einem Buch P1, PP.

Figur 4 zeigt einen Schnitt durch ein Produkt P1, das eine Aufnahmeöffnung OP1 für ein Trägermedium CA aufweist. Das Trägermedium CA ist dabei in der Weise in die Aufnahmeöffnung integriert, daß das Abdeckungsteil CO des Trägermediums frei zugänglich ist und abgelöst werden kann. Das Trägermedium kann an seiner Unterseite ein Klebemittel aufweisen und lösbar mit der Aufnahmeöffnung OP1 verbunden sein

Figur 5 zeigt ein erfindungsgemäßes Trägermedium CA für mindestens einen ersten Duftstoff D1, das zwischen einer Verpackung PA und dem verpackten Produkt P1 angeordnet ist. Die Verpackung PA ist mit dem Abdeckungsteil CO des Trägermediums verbunden, während das Basisteil BA des Trägermediums an seiner Unterseite mit dem Produkt P1 verbunden ist. Das Abdeckungsteil CO des Trägermedium ist in der Weise mit der Verpackung PA verbunden, daß bei einem Öffnen der Verpackung PA die Abdeckung CO gelöst wird und der Duftstoff freigesetzt wird.

Figur 6 zeigt ein erfindungsgemäßes Trägermedium CA für mindestens einen ersten Duftstoff D1, das zwischen einer ersten Seite (oder einem Buchdeckel) PG1 und einer Seite PG2 eines Buchs P1, PP angeordnet ist.

Die Seite PG1 ist mit dem Abdeckungsteil CO des Trägermediums verbunden, während das Basisteil BA des Trägermediums an seiner Unterseite mit der Seite PG2 verbunden ist. Das Abdeckungsteil CO des Trägermedium ist in der Weise mit der Seite PG1 verbunden, daß bei einem Umblättern von Seite PG1 zu PG2 bzw. bei einem Öffnen des Buches die Abdeckung CO gelöst wird und der Duftstoff freigesetzt wird.

Als erstes Produkt P1 kann auch ein Produkt wie zum Beispiel ein Kugelschreiber vorgesehen sein, das mindestens ein bewegliches Teil aufweist. Das Trägermedium CA ist bei dieser Ausführungsform in der Weise dem ersten Produkt räumlich zugeordnet, daß durch eine Bewegung des beweglichen Teil mindestens der erste Duftstoff D1 freigesetzt wird.

Ebenso kann das Trägermedium CA in der Weise an dem ersten Produkt P1 angeordnet sein, daß durch äußere Krafteinwirkung auf das erste Produkt P1 oder durch von dem ersten Produkt P1 freigesetzte Kraft mindestens der erste Duftstoff D1 freigesetzt wird.

Beispielsweise ist das erste Produkt P1 eine Dateneingabeeinrichtung wie eine Taste, ein Bedienknopf, eine sogenannte Mouse, und/oder ein der Dateneingabeeinrichtung räumlich zugeordneter Gegenstand, wie beispielsweise eine Unterlage für eine Mouse (mouse pad). Ein weiteres Beispiel eines ersten Produkts P1 ist ein Stempel, der so ausgestaltet ist, daß bei Druck des Stempels gegen einen zu stempelnden Gegenstand ebenfalls Druck auf Trägermaterial MA (Fig. 1) ausgeübt wird. Durch den Druck des Stempels gegen den zu stempelnden Gegenstand kann auch ein Abdeckungsteil CO (Figuren 5, 6) von dem Trägermedium gelöst werden, so daß ein Duftstoff freigesetzt wird.

Das erste Produkt P1 kann auch ein mit Hand und/oder mit Fuß betätigbares Produkt sein, ein Handgriff, ein Pedal, ein Kissen, ein Bodenbelag (z. B. Fußmatte), ein Lüftungsrad (z.B. in einer Datenverarbeitungseinrichtung; Ventilator) oder ein Musikinstrument.

Das Trägermedium CA kann auch in der Weise an dem ersten Produkt P1 angeordnet sein, daß durch Wärme, die dem ersten Produkt P1 zugeführt wird oder die das erste Produkt P1 selbst erzeugt, der erste Duftstoff D1 freigesetzt wird. Insbesondere ist bei dieser Ausführungsform das erste Produkt P1 ein Produkt, das elektrische Energie aufnimmt, wie beispielsweise ein elektrisches Bauteil (Leistungshalbleiter, Transformator).

Das Trägermedium CA kann auch in einem vierten Produkt P4 integriert sein. Ein Beispiel für das vierte Produkt P4 ist ein Behälter.

Der Behälter kann mindestens ein erstes Trägermedium CA1 und ein zweites Trägermedium CA2 aufweisen und in der Weise mit mindestens einem beweglichen Teil ausgestaltet sein, daß sich der Behälter trägermediumindividuell öffnen und schließen läßt Ein solcher Behälter ist in Figur 7 in Seitenansicht und in Draufsicht dargestellt Der Behälter hat die Form eines Zylinders und ist zum Beispiel durch zwei Deckel C1 und C2 abgedeckt. In dem Behälter sind zum Beispiel 16 Trägermedien CA angeordnet, wobei 8 Trägermedien CA1 bis CA8 dem Behälterdeckel C1 zugewandt sind C1 weist 8 kreisförmige Öffnungen auf, wobei jeweils eine Öffnung über einem Trägermedium positionierbar ist. Der Deckel C1 läßt sich darüber hinaus in einer Position verriegeln, in der keine der 8 Öffnungen ein Trägermedium freigibt. Der Behälterdeckel C2 kann wie der Behälterdeckel C1 ausgestaltet sein.

Das vierte Produkt P4 ist z.B. ein sogenannter Hand- oder Fußschmeichler und/oder eine deformierbare Masse, wie beispielsweise Wachs oder Knetgummi.

Das erfindungsgemäße Trägermedium läßt sich in verschiedenen Branchen einsetzen, zum Beispiel im Verlagswesen, im Unterhaltungsbereich, in der Gastronomie, in der Touristikbranche, als Trägermedium für unternehmensindividuelle Duftstoffe, im Bereich des Versandhandels, im Marketing unterschiedlicher Waren und Dienstleistungen einschließlich im Bereich Immobilien, Versicherungen und Personalmanagement.

Figur 8 zeigt den Aufbau eines Trägermediums CA nach dem Stand der Technik. Dieses besteht aus einem Basisteil BA und aus einem Abdeckungsteil CO. Basisteil BA und Abdeckungsteil CO umschließen einen Duftstoff D1 und sind miteinander verklebt.

### Bezugszeichenliste

- CA: Trägermedium
- CA1, ..., CA8: erstes/zweites Trägermedium
- MA: flüssigkeitsspeicherndes Material
- BA: Basisteil von CA
- CO: Abdeckungsteil von CA
- CP1, CP2: Klebebereiche von CA
- BE1, BE2: Begrenzungselemente von CA
- D1: erster Duftstoff
- D2: zweiter Duftstoff
- P1,...P4: erstes, ... viertes Produkt
- PP: Druckerzeugnis
- PG1, PG2: Elemente von PP
- IM: Informationsspeicher
- OP1: Aufnahmeöffnung für CA

## Patentansprüche

1. Trägermedium (CA) für mindestens einen ersten Duftstoff (D1), wobei das Trägermedium (CA) mit einem ersten Produkt (P1) verbindbar ist, und wobei das Trägermedium mindestens den ersten Duftstoff (D1) enthält,
**dadurch gekennzeichnet**,
daß das erste Produkt (P1) eine Aufnahmeöffnung (OP1) für das Trägermedium (CA) aufweist.

2. Trägermedium nach Anspruch 1, dadurch gekennzeichnet, daß das erste Produkt (P1) ein Druckerzeugnis (PP), ein elektrischer und/oder magnetischer Informationsspeicher (IM) und/oder eine Verpackung (PA) ist.

3. Trägermedium nach einem der vorstehenden Ansprüche, daß der erste Duftstoff (D1) ein natürlicher Duftstoff ist.

4. Trägermedium nach Anspruch 3, dadurch gekennzeichnet, daß der erste Duftstoff (D1) ein ätherisches Öl ist.

5. Trägermedium nach Anspruch 1 oder 2, dadurch gekennzeichnet, daß der erste Duftstoff (D1) synthetisch ist.

6. Trägermedium nach einem der Ansprüche 2 bis 5, dadurch gekennzeichnet, daß das Druckerzeugnis (PP) ein zweites Produkt (P2) und/oder ein drittes Produkt (P3) beschreibt, das den ersten Duftstoff (D1) und/oder einen zweiten Duftstoff (D2) enthält, der mindestens eine Duftkomponente des ersten Duftstoffs (D1) aufweist.

7. Trägermedium nach einem der Ansprüche 2 bis 6, dadurch gekennzeichnet, daß der Informationsspeicher (IM) Daten zu einem dritten Produkt (P3) aufweist, das neben einem oder mehreren Stoffen den ersten Duftstoff (D1) und/oder einen zweiten Duftstoff (D2) enthalt, der mindestens eine Duftkomponente des ersten Duftstoffs (D1) enthält.

8. Trägermedium nach einem der vorstehenden Ansprüche, dadurch gekennzeichnet, daß das Trägermedium (CA) flüssigkeitspeicherndes Material (MA) aufweist.

9. Trägermedium nach einem der vorstehenden Ansprüche, dadurch gekennzeichnet, daß das Trägermedium (CA) mit dem ersten Duftstoff (D1) und/oder mit einem zweiten Duftstoff (D2) wiederbefüllbar ist

10. Trägermedium nach einem der vorstehenden Ansprüche, dadurch gekennzeichnet daß das Trägermedium (CA) eine lösbar angeordnete Abdeckung (CO) aufweist, und daß die lösbar angeordnete Abdeckung (CO) in der Weise mit einer Verpackung (PA) des ersten Produkts (P1) verbunden ist, daß bei einem Öffnen der Verpackung (PA) die Abdeckung (CO) gelöst wird.

11. Trägermedium nach einem der Ansprüche 2 bis 10, dadurch gekennzeichnet, daß das Druckerzeugnis (PP) mindestens zwei aufeinander angeordnete Elemente (PG1, PG2) aufweist, daß das Trägermedium (CA) eine lösbar angeordnete Abdeckung (CO) aufweist, und daß die lösbar angeordnete Abdeckung (CO) in der Weise mit den aufeinander angeordneten Elementen (PG1, PG2) des Druckerzeugnisses (PP) verbunden ist, daß bei einem Trennen der beiden aufeinander angeordneten Elemente (PG1, PG2) des Druckerzeugnisses die Abdeckung (CO) des Trägermediums (CA) gelöst wird.

12. Trägermedium nach einem der vorstehenden Ansprüche, dadurch gekennzeichnet, daß das erste Produkt (P1) mindestens ein bewegliches Teil aufweist und daß das Trägermedium (CA) in der Weise dem ersten Produkt räumlich zugeordnet ist, daß durch eine Bewegung des beweglichen Teil mindestens der erste Duftstoff (D1) freigesetzt wird.

13. Trägermedium nach einem der vorstehenden Ansprüche, dadurch gekennzeichnet, daß das Trägermedium (CA) einen selbstklebenden Bereich aufweist, der mit dem ersten Produkt (P1) verbindbar ist.

14. Trägermedium nach einem der vorstehenden Ansprüche, dadurch gekennzeichnet, daß das Trägermedium (CA) in der Weise an dem ersten Produkt (P1) angeordnet ist, daß durch äußere Krafteinwirkung auf das erste Produkt (P1) oder durch von dem ersten Produkt (P1) freigesetzte Kraft mindestens der erste Duftstoff (D1) freigesetzt wird.

15. Trägermedium nach Anspruch 14, dadurch gekennzeichnet daß das erste Produkt (P1) eine Dateneingabeeinrichtung und/oder ein der Dateneingabeeinrichtung räumlich zugeordneter Gegenstand ist.

16. Trägermedium nach Anspruch 14, dadurch gekennzeichnet, daß das erste Produkt (P1) ein mit Hand und/oder mit Fuß betätigbares Produkt, ein Handgriff, ein Pedal, ein Kissen, ein Bodenbelag, ein Lüftungsrad oder ein Musikinstrument ist.

17. Trägermedium nach einem der vorstehenden Ansprüche, dadurch gekennzeichnet, daß das Trägermedium (CA) in der Weise an dem ersten Produkt (P1) angeordnet ist, daß durch dem ersten Produkt (P1) zugeführte oder durch von dem ersten Produkt (P1) freigesetzte Wärme mindestens der erste Duftstoff (D1) freigesetzt wird.

18. Trägermedium nach Anspruch 17, dadurch gekennzeichnet, daß das erste Produkt (P1) ein Produkt ist, das elektrische Energie aufnimmt.

19. Trägermedium nach Anspruch 17 oder 18, dadurch gekennzeichnet, daß das erste Produkt (P1) eine elektrisches Bauteil ist.

20. Trägermedium (CA) für mindestens einen ersten Duftstoff (D1),
**dadurch gekennzeichnet**,
daß das Trägermedium (CA) in ein viertes Produkt (P4) integriert ist.

21. Trägermedium (CA) für mindestens einen ersten Duftstoff (D1),
**dadurch gekennzeichnet**,
daß das vierte Produkt (P4) ein Behälter ist.

22. Trägermedium (CA) nach Anspruch 21, dadurch gekennzeichnet, daß der Behälter mindestens ein erstes Trägermedium (CA1) und ein zweites Trägermedium (CA2) aufweist und daß der Behälter in der Weise mit mindestens einem beweglichen Teil ausgestaltet ist, daß sich der Behälter trägermediumindividuell öffnen und schließen läßt

23. Trägermedium nach Anspruch 20, dadurch gekennzeichnet, daß das vierte Produkt (P4) ein Hand- oder Fußschmeichler und/oder eine deformierbare Masse ist.

24. Trägermedium nach Anspruch 25, dadurch gekennzeichnet, daß die deformierbare Masse Wachs oder Knetgummi ist.

25. Trägermedium nach einem der vorstehenden Ansprüche, dadurch gekennzeichnet, daß das erste Produkt (P1) eine Dienstleistung oder ein Objekt beschreibt.
